Europäisches Patentamt

European Patent Office

Office européen des brevets

(19)

(11) Veröffentlichungsnummer: **0 084 684**
**B1**

(12) # EUROPÄISCHE PATENTSCHRIFT

(45) Veröffentlichungstag der Patentschrift:
10.04.85

(21) Anmeldenummer: 82112129.0

(22) Anmeldetag: 31.12.82

(51) Int. Cl.⁴: **C 12 N 9/96, C 12 Q 1/60**

(54) **Verfahren zur Stabilisierung wässriger Lösungen von Cholesterinesterase aus Pseudomonaden.**

(30) Priorität: 07.01.82 DE 3200274

(43) Veröffentlichungstag der Anmeldung:
03.08.83 Patentblatt 83/31

(45) Bekanntmachung des Hinweises auf die Patenterteilung:
10.04.85 Patentblatt 85/15

(84) Benannte Vertragsstaaten:
AT BE CH DE FR GB IT LI LU NL SE

(56) Entgegenhaltungen:
EP - A - 0 016 946
FR - A - 891 187
US - A - 3 884 764

ENZYME MICROBIAL TECHNOLOGY, Band 1, April
1979, Selten 74-82, IPC Business Press, Sussex, G.B.
V.P. TORCHILIN: "Enzyme stabilization without
carriers"
AGRICULTURAL AND BIOLOGICAL CHEMISTRY, Band
40, Nr. 10, Oktober 1976, Selten 1957-1964 T. UWAJIMA
et al.: "Purification and properties of cholesterol
esterase from Pseudomonas fluorescens"

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Buschek, Herbert, Paradeisstrasse 36,
D-8120 Weilheim (DE)
Erfinder: Schlumberger, Helmut,
Kalser-Heinrich-Strasse 23, D-8121 Polling (DE)
Erfinder: Siedel, Joachim, Dr. rer. nat.,
Bahnhofstrasse 6, D-8131 Bernried (DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)

## Beschreibung

Die Erfindung betrifft ein Verfahren zur Stabilisierung einer Cholesterinesterhydrolase (Cholesterinesterase, E.C. 3.1.1.13) aus Pseudomonaden in wässeriger, gegebenenfalls ein oberflächenaktives Mittel enthaltender Lösung.

Cholesterinesterasen werden in erheblichem Umfang in Reagenzien zur enzymatischen Analyse cholesterinesterhaltiger Lösungen, insbesondere von Serum in der klinischen Diagnostik, eingesetzt.

Diese Enzyme bewirken dabei die Freisetzung von Cholesterin aus seinen Estern mit langkettigen Fettsäuren, welches anschliessend mit geeigneten Nachweisverfahren, gewöhnlich enzymatisch unter Verwendung der durch die Cholesterinoxidase (E.C. 1.1.3.6.) katalysierten Reaktion, bestimmt wird.

Für die Bestimmung dieses veresterten Cholesterins im Serum eignet sich insbesondere Cholesterinesterase aus Pseudomonaden, da diese auch eine Lipaseaktivität aufweist. Ein Enzym mit dieser Eigenschaft ist z.B. in DAS Nr. 2819384 beschrieben. Derartige Enzyme sind in der Lage, die häufig durch den Triglyceridgehalt des Probenmaterials im Reaktionsansatz hervorgerufenen Trübungen zu beseitigen (aufzuklaren) und dadurch z.B. Störungen bei photometrischen Messverfahren auszuschalten.

Solche Cholesterinesterasen mit Lipaseaktivität können auch zum Nachweis von Triglyceriden im Probenmaterial eingesetzt werden, wenn das freiwerdende Glycerin einer geeigneten Nachweisreaktion unterworfen wird.

Darüber hinaus können Enzyme dieser Art allgemein in Reagenzien zur Serumanalyse Verwendung finden, mit denen zwar weder Cholesterinester noch Triglyceride bestimmt werden, bei denen aber aus messtechnischen Gründen eine Beseitigung der durch die Triglyceride aus dem Probenmaterial verursachten Trübungen wünschenswert ist.

Es ist nun bekannt, dass Cholesterinesterase in einem gepufferten Medium, das keine weiteren Komponenten enthält, gegenüber ihren Substraten keine oder nur eine sehr geringe hydrolytische Aktivität entfalten. Es ist daher erforderlich, dem Reaktionsgemisch Aktivatoren hinzuzufügen.

Als Aktivatoren sind viele oberflächenaktive Substanzen (Detergenzien) geeignet, wie nichtionische Detergenzien, z.B. Alkyl- bzw. Aryl- und Aralkylalkoholpolyglykoläther (®Triton X-100, ®Thesit, ®Lutensol ON 60 bzw. 70, Isotridecyläther), und/oder anionische Detergenzien, z.B. Salze der Gallensäuren oder ihrer Konjugate.

Die aktivierende Wirkung wird häufig durch eine erhöhte Ionenstärke im Reaktionsmedium unterstützt, wobei die optimale Ionenstärke am einfachsten durch eine entsprechende Dosierung der Puffersubstanzen erzeugt wird.

Als Puffer eignen sich diejenigen, die im Bereich des Aktivitätsoptimums der Cholesterinesterasen, meist also zwischen pH 5 und pH 9, am wirksamsten sind. Besonders bevorzugt werden Phosphatpuffer, wie aus dem über Cholesterinesterasen vorhandenen Literaturmaterial hervorgeht.

Als Cholesterinesterasen werden meist Enzyme tierischer oder mikrobieller Herkunft eingesetzt, wobei allerdings zwischen den verschiedenen Enzymen z.T. erhebliche Unterschiede im Wirkungsspektrum gegenüber der Palette der im Serum auftretenden Cholesterinfettsäureester bestehen können. Vahouny, Weersing und Treadwell („Arch. Biochem. Biophys." (1964), 107, 7-15) beschreiben eine Cholesterinesterase aus Pankreassaft, deren enzymatische Aktivität von der Art und der Kettenlänge des Fettsäureesters weitgehend unbeeinflusst bleibt, während aus „Z. Klin. Chem. Klin. Biochem." (1974), 12, 403-407, eine mikrobielle Cholesterinesterase bekannt ist, deren hydrolytische Wirksamkeit relativ stark von der Natur des Fettsäureanteils im Cholesterinester abhängt.

Weiterhin existieren Cholesterinesterasen, die Triglyceride nicht oder nur mit geringer Geschwindigkeit umzusetzen vermögen, wie z.B. in „J. Biol. Chem." (1962), 237, 3469-3656, beschrieben wird.

Die oben erwähnte Cholesterinesterase aus Pseudomonaden weist bezüglich der Art des Fettsäureanteils im Cholesterinester ein sehr breites Wirkungsspektrum auf, setzt gleichzeitig auch Triglyceride mit hoher Geschwindigkeit unter geeigneten Reaktionsbedingungen um und wäre daher aus den anfangs genannten Gründen ganz besonders für die Herstellung von Reagenzien zur Analyse cholesterinester- und/oder triglyceridhaltiger Lösungen, wie z.B. Serum, geeignet.

Die Verwendung des Enzyms für diese Zwecke setzt allerdings voraus, dass es im gebrauchsfertigen Reagenz, d.h. in wässeriger Lösung, hinreichend stabil ist.

Es wurde nun gefunden, dass das Enzym aus Pseudomonaden zwar in reinem Phosphatpuffer, der gewöhnlich für die Herstellung handelsüblicher Reagenzien zur Bestimmung des Serumgesamtcholesterins verwendet wird, eine noch ausreichende Stabilität besitzt, jedoch überraschenderweise schnell seine Aktivität verliert, wenn in der Phosphatpufferlösung ein oberflächenaktives Mittel der oben genannten Art, also z.B. ®Triton X-100 oder Isotridecyläther, als Aktivator enthalten ist. Es kann demnach nicht ohne weiteres in Reagenzlösungen eingesetzt werden, von denen aus Praktikabilitäts- und Kostengründen eine ausreichende, mindestens dem Stand der Technik bei Cholesterinesterasen anderer Herkunft entsprechende Lagerfähigkeit gefordert wird.

Der Erfindung liegt daher die Aufgabe zugrunde, diesen erheblichen Nachteil zu beseitigen und ein Verfahren zu schaffen, mit dessen Hilfe die Cholesterinesterase so stabilisiert wird, dass sie im gebrauchsfertigen Reagenz über einen längeren Zeitraum, mindestens 3 bis 5 d, unter Raumtemperaturbedingungen, ohne wesentlichen Aktivitätsverlust aufbewahrt werden kann.

Gelöst wird die Aufgabe erfindungsgemäss durch ein Verfahren zur Stabilisierung wässeriger Lösungen von Cholesterinesterase aus Pseudomonaden, insbesondere in Gegenwart eines oberflächenaktiven Mittels, welches dadurch gekennzeichnet ist, dass das Enzym in einem phosphatfreien Puffer gelöst wird, welcher 10 bis 200 mmol pro Liter Magnesiumionen enthält.

Vorzugsweise stellt man die Konzentration der Magnesiumionen zwischen 25 und 150, besonders bevorzugt zwischen 50 und 100 mmol/l ein.

Als Puffer kommen Substanzen wie Tris/ Tris·HCl, Triäthanolamin/Triäthanolamin·HCl, Imidazol, Hepes, Mops und andere phosphatfreie Puffergemische in betracht. Bevorzugt wird Trispuffer eingesetzt. Der pH-Wert der Pufferlösung liegt zwischen 5,0 und 9,0, bevorzugt zwischen 6,5 und 9,0, besonders bevorzugt zwischen 7,5 und 8,5 insbesondere, wenn Trispuffer verwendet wird.

Der instabilisierende Effekt von Phosphat beim Pseudomonadenenzym, insbesondere in Gegenwart oberflächenaktiver Mittel, ist überraschend und war aus dem über Cholesterinesterasen vorliegenden Literaturmaterial nicht abzuleiten.

So wird in „J. Biol. Chem." (1957), *228*, 447-457, eine Cholesterinesterase aus Schweinepankreas beschrieben, deren enzymatische Aktivität in Phosphatpuffer mit Taurocholat als Enzymaktivator gemessen wird. Ein nachteiliger Einfluss des Phosphatpuffers auf die Eigenschaften des Enzyms wird in dieser Arbeit nicht erwähnt.

Weiterhin finden sich Angaben über Eigenschaften von Cholesterinesterasen in vielen Literaturstellen, wie z.B. in „J. Biochem." (Jap.) (1974), *75*, 1073-1079, „Biochim. Biophys. Acta" (1971), *231*, 194-197, „Arch. Biochem. Biophys." (1963), *100*, 360-363, „Clin. Chem." (1974), *20*, 470-475, sowie „Biochim. Biophys. Acta" (1975), *384*, 138-145; in allen genannten Arbeiten erfolgt die Bestimmung der Enzymaktivität in phosphatpufferhaltigen Lösungen, die zum Teil oberflächenaktive Stoffe enthalten, wobei weder eine instabilisierende Wirkung des Phosphats noch eine stabilisierende Wirkung von Magnesiumsalzen auf die Cholesterinesterasen offenbart wird.

Entsprechendes gilt für die Literaturstellen, die sich mit mikrobiellen Cholesterinesterasen befassen.

Die Verwendung einer mikrobiellen Cholesterinesterase in einem Reagenz zur vollenzymatischen Bestimmung des Serumcholesterins ist schliesslich in „Z. Klin. Chem. Klin. Biochem." (1974), *12*, 403-407, beschrieben. Das Reagenz enthält neben ®Thesit als Detergens eine verhältnismässig hohe Konzentration an Ammoniumphosphatpuffer, wobei auf eine gute Stabilität des die Esterase enthaltenden Reagenzes besonders hingewiesen wird.

Die Eigenschaften einer Cholesterinesterase aus *Pseudomonas fluoreszenz* finden sich in „Agric. Biol. Chem." (1975), *39*, 1511-1512. Die Aktivität des Enzyms wird in Anlehnung an die in „Clin. Chem." (1974), *20*, 470-475, beschriebene Methode bestimmt, wobei das Reagenz auch in diesem Fall mit Phosphat gepuffert wird.

Eine destabilisierende Wirkung von Phosphationen auf die Cholesterinesterase aus Pseudomonaden bei Gegenwart von oberflächenaktiven Mitteln war daher nicht bekannt, so dass auch kein Anlass bestand, Phosphationen aus der wässerigen Lösung derartiger Cholesterinesterasen fernzuhalten. Ebensowenig war vorhersehbar gewesen, dass dieser Effekt durch die Zugabe von Magnesiumionen im angegebenen Konzentrationsbereich beseitigt werden kann.

Die Erfindung ist zwar von besonderer Bedeutung für die Stabilisierung von Cholesterinesterasen aus Pseudomonaden in Lösungen, welche auch ein oberflächenaktives Mittel enthalten, jedoch lässt sich erfindungsgemäss eine bessere Erhaltung der Aktivität dieses Enzyms in wässeriger Lösung auch dann erzielen, wenn kein oberflächenaktives Mittel zugegen ist.

Cholesterinesterase aus Pseudomonaden ist bekannt und handelsüblich. Es wurde bereits in vielen verschiedenen Stämmen von Pseudomonaden gefunden, beispielsweise in *Pseudomonas fluoreszenz* (DE-OS Nr. 2819384) oder *Pseudomonas sp.* (DE-OS Nr. 2933646). Bei allen untersuchten Cholesterinesterasepräparaten aus Pseudomonaden erwies sich das erfindungsgemässe Verfahren als sehr wirksam, unabhängig davon, welcher Cholesterinesterase enthaltende Pseudomonadenstamm als Quelle für das Enzym verwendet wurde. Dies geht aus Versuchen hervor, die mit *Pseudomonas sp.* DSM 1280, handelsüblicher Cholesterinesterase aus *Pseudomonas fluoreszenz* (Sigma, Kat. Nr. C 2770) und *Pseudomonas sp.* DMS 1281-Cholesterinesterase durchgeführt wurden. Die Cholesterinesterase aus diesen drei Pseudomonasstämmen wurde jeweils in einer Lösung untersucht, die als oberflächenaktives Mittel 10 mmol Natriumcholat und 0,3% Polyäthoxyfettalkoholäther enthielt. Die Ergebnisse zeigt die beigefügte Zeichnung.

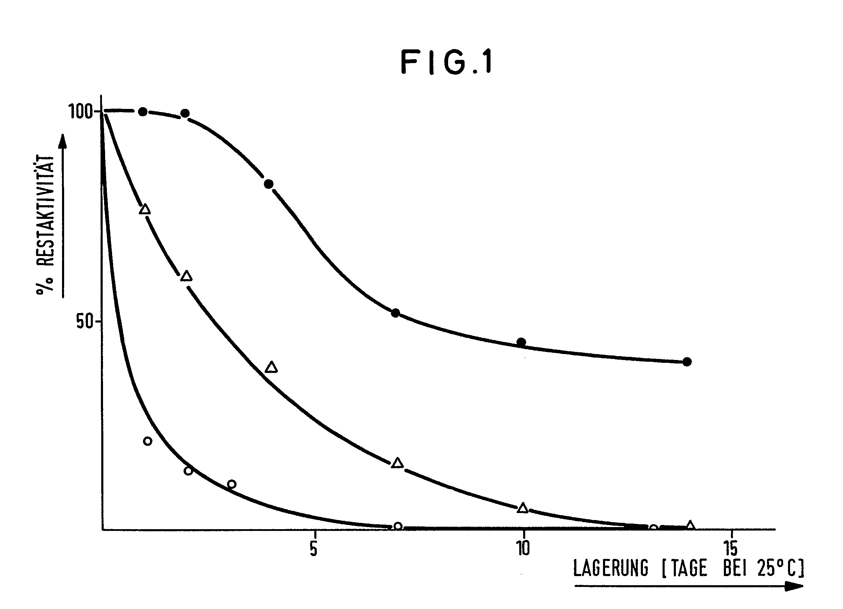

Fig. 1 stellt eine graphische Darstellung dar, in der die prozentuale Aktivität während der Lagerung bei 25°C aufgetragen ist. Die durch Kreise gebildete Kurve bezieht sich auf die Enzymlösung in K-Phosphatpuffer 0,1 mol/l pH 7,6; die durch Dreiecke gebildete Kurve bezieht sich auf Enzym in Tris·HCl-Puffer 0,1 mol/l pH 7,0 und die durch schwarze Punkte gekennzeichnete Kurve auf Tris·HCl 0,1 mol/l pH 7,6 enthaltend 50 mmol pro Liter Magnesiumaspartat.

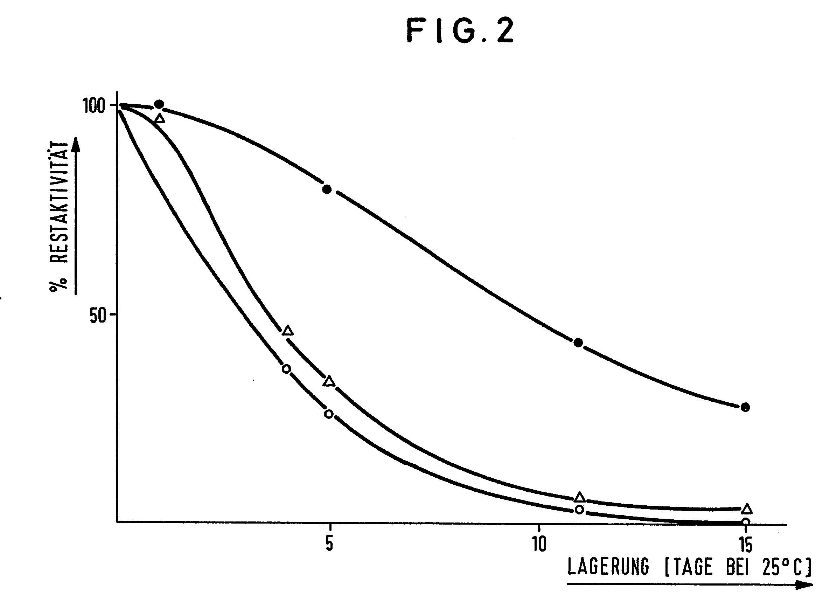

Fig. 2 entspricht Fig. 1, jedoch für handelsübliche Esterase aus *Pseudomonas fluoreszenz.*

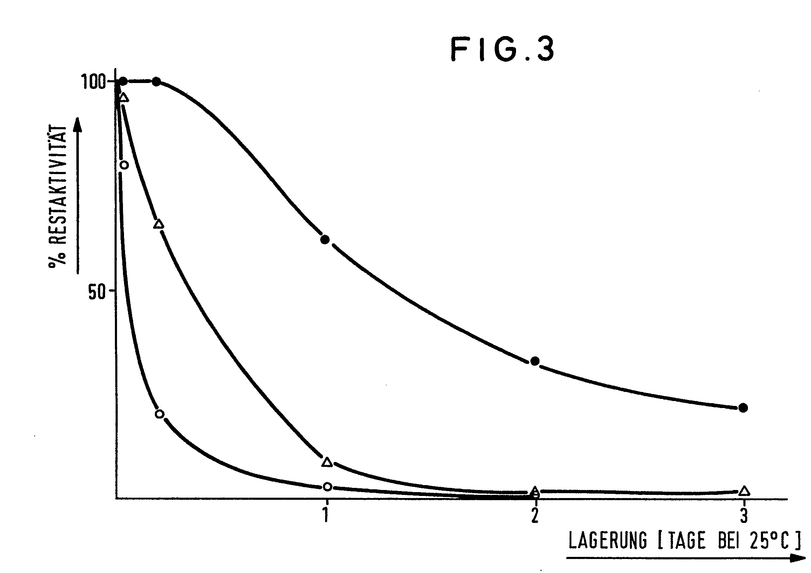

Fig. 3 entspricht Fig. 1, jedoch für Cholesterinesterase aus *Pseudomonas sp.* DSM 1281.

Die Magnesiumionen können in Form beliebiger Magnesiumsalze zugesetzt werden, deren Anion keine nachteilige Einwirkung auf irgendwelche Bestandteile der Enzymlösung ausübt. Neben den Magnesiumsalzen der anorganischen Säuren, wie Magnesiumchlorid oder Magnesiumsulfat, sind vor allem die Magnesiumsalze organischer Säuren, wie der Fettsäuren, Dicarbonsäuren und Aminosäuren geeignet. Bevorzugt werden die Magnesiumsalze von Aminosäuren, insbesondere Magnesiumaspartat.

Bei der stabilisierenden Wirkung der Magnesiumionen auf die Cholesterinesterase aus Pseudomonaden handelt es sich nicht um eine aktivierende Wirkung. Hierfür spricht, dass in einer ein oberflächenaktives Mittel enthaltenden Lösung hinsichtlich der Aktivität kein Unterschied besteht, ob man Magnesiumaspartat oder Kochsalz in vergleichbarer Konzentration zusetzt, wobei letzteres keine stabilisierende Wirkung besitzt. Weiter spricht hierfür, dass eine durch Lagerung in detergenshaltigem Phosphatpuffer weitgehend inaktivierte Cholesterinesterase aus Pseudomonaden durch nachträglichen Zusatz von Magnesiumsalzen auch in höheren Konzentrationen, d.h. bis zur Löslichkeitsgrenze in Phosphatpuffer, nicht reaktiviert wird.

Durch die Erfindung gelingt es, die aufgrund ihrer Eigenschaften für die Bestimmung des veresterten Cholesterins besonders geeignete Cholesterinesterase aus Pseudomonaden in wässeriger Lösung so zu stabilisieren, dass die angestreibte Haltbarkeit erzielt wird.

Die erfindungsgemäss erzielte verbesserte Stabilität wird durch die nachstehenden Beispiele weiter veranschaulicht. In diesen werden folgende Abkürzungen verwendet:

Che = Cholesterinesterase aus Pseudomonaden

| | |
|---|---|
| Hepes | = n-2-Hydroxyäthylpiperazin-n'-äthansulfonsäure |
| Tris | = Tris(hydroxymethyl)amino-methan |
| Isotridecyläther | = Polyoxyäthylenisotridecyläther |
| " Thesit | = Polyoxyäthylendodecyläther |
| Mops | = 3-(n-Morpholino)propansulfonsäure |
| " Triton X-100 | = Polyoxyäthylenisooctyl-phenyläther |
| " Lutensol ON 60 bzw. ON 70 | = Polyoxyäthylenfettalkohol-äther |

*Beispiel 1*

Die Stabilität der Che wurde bei 25 °C in verschiedenen Puffern mit und ohne Zusatz von $MG^{2+}$ untersucht.

Tabelle 1 zeigt die mit vier verschiedenen Puffern und zwei verschiedenen oberflächenaktiven Mitteln ohne Magnesiumzusatz erzielten Resultate, Tabelle 2 die entsprechenden Ergebnisse bei 50 mmol $Mg^{2+}$-Zusatz. In allen Fällen wurde 10 mmol pro Liter Cholat und 0,3% des jeweils angegebenen nichtionischen oberflächenaktiven Mittels eingesetzt.

*Tabelle 1*

| Nichtionisches oberflächenaktives Mittel | Puffer (100 mmol) | Che-Stabilität (%) | | | | | |
|---|---|---|---|---|---|---|---|
| | | 1d | 2d | 4d | 7d | 10d | 14d |
| Isotridecyläther | K-Phosphat pH = 7,6 | 21 | 14 | 11 | 0 | | |
| | Tris pH = 7,6 | 77 | 61 | 39 | 16 | 5 | 0 |
| | Imidazol pH = 7,6 | 78 | 72 | 50 | 10 | 8 | 6 |
| | Mops pH = 7,0 | 87 | 74 | 43 | 46 | 33 | 27 |
| " Triton X-100 | K-Phosphat | 2 | 0 | | | | |
| | Tris | 58 | 34 | 0 | | | |
| | Imidazol | 57 | 35 | 10 | 2 | 0 | |
| | Mops | 76 | 53 | 20 | 9 | 7 | 0 |

*Tabelle 2*

| Nichtionisches oberflächenaktives Mittel | Puffer | Che-Stabilität (%) | | | | | |
|---|---|---|---|---|---|---|---|
| | | 1 d | 2 d | 4 d | 7 d | 10 d | 14 d |
| Isotridecyläther | Tris pH = 7,6 | 102 | 102 | 83 | 52 | 45 | 40 |
| | Imidazol pH = 7,6 | 84 | 87 | 71 | 55 | 43 | 38 |
| | Mops pH = 7,0 | 96 | 82 | 63 | 61 | 58 | 56 |

*Tabelle 2 (Fortsetzung)*

| Nichtionisches oberflächenaktives Mittel | Puffer | Che-Stabilität (%) | | | | | |
|---|---|---|---|---|---|---|---|
| | | 1 d | 2 d | 4 d | 7 d | 10 d | 14 d |
| ®Triton X-100 | Tris | 85 | 77 | 50 | 36 | 35 | 30 |
| | Imidazol | 75 | 60 | 33 | 25 | 30 | 20 |
| | Mops | 92 | 82 | 53 | 51 | 45 | 34 |

Die obigen Ergebnisse zeigen, dass bei der erfindungsgemässen Kombination von phosphatfreiem Puffer und Magnesiumionen die Stabilität wesentlich verbessert wird.

*Beispiel 2*

Die Abhängigkeit der stabilisierenden Wirkung von der Magnesiumkonzentration wurde bei 25 °C in 100 mmol Trispuffer pH 7,6 untersucht. Die erhaltenen Ergebnisse zeigt Tabelle 3. Bezüglich der oberflächenaktiven Mittel gelten die Angaben von Beispiel 1.

*Tabelle 3*

| Nichtionisches oberflächenaktives Mittel | Mg²⁺-Konz. (mmol) | Che-Stabilität (%) | | | | | |
|---|---|---|---|---|---|---|---|
| | | 1 d | 2 d | 4 d | 7 d | 10 d | 14 d |
| Isotridecyläther | 0 | 77 | 61 | 39 | 16 | 5 | 0 |
| | 10 | 102 | 94 | 81 | 53 | 33 | 33 |
| | 50 | 102 | 102 | 83 | 52 | 45 | 40 |
| | 100 | 98 | 92 | 79 | 64 | 56 | 49 |
| | 150 | 104 | 102 | 93 | 68 | 61 | 56 |
| ®Triton X-100 | 0 | 58 | 34 | 0 | | | |
| | 30 | 65 | 51 | 17 | 19 | 10 | 7 |
| | 50 | 85 | 77 | 50 | 36 | 35 | 30 |
| | 100 | 93 | 85 | 66 | 43 | 35 | 32 |
| | 150 | 96 | 81 | 35 | 34 | 31 | 30 |

Die in Tabelle 3 gezeigten Ergebnisse lassen erkennen, dass bereits bei einer Mg²⁺-Konzentration von 10 mmol die Stabilität wesentlich erhöht wird.

*Beispiel 3*

Wie in Beispiel 1 beschrieben, wurde die Stabilität der Che mit und ohne Magnesiumionenzusatz bei verschiedenen Puffern und verschiedenen oberflächenaktiven Mitteln untersucht. Im Unterschied zu Beispiel 1 wurde die Lösung jedoch bei 4 °C gehalten.

Tabelle 4 zeigt die Stabilität ohne Magnesium, Tabelle 5 mit 50 mmol Mg²⁺.

*Tabelle 4*

| Nichtionisches oberflächenaktives Mittel | Puffer | Che-Stabilität (%) | | | | | | | |
|---|---|---|---|---|---|---|---|---|---|
| | | 2 d | 4 d | 7 d | 14 d | 21 d | 28 d | 35 d | 42 d |
| Isotridecyläther | K-Phosphat pH = 7,6 | 35 | 13 | 2 | 0 | | | | |
| | Tris pH = 7,6 | 93 | 63 | 54 | 48 | 32 | 28 | 21 | 18 |
| | Imidazol pH = 7,6 | 85 | 60 | 57 | 50 | 37 | 28 | 28 | 22 |
| | Mops pH = 7,0 | 81 | 60 | 58 | 60 | 60 | 55 | 47 | 40 |

*Tabelle 4 (Fortsetzung)*

| Nichtionisches oberflächenaktives Mittel | Puffer | Che-Stabilität (%) | | | | | | | |
|---|---|---|---|---|---|---|---|---|---|
| | | 2 d | 4 d | 7 d | 14 d | 21 d | 28 d | 35 d | 42 d |
| ®Triton X-100 | K-Phosphat | 29 | 13 | 13 | 8 | 0 | | | |
| | Tris | 58 | 35 | 31 | 25 | 13 | 6 | 2 | 0 |
| | Imidazol | 65 | 43 | 35 | 35 | 35 | 30 | 24 | 20 |
| | Mops | 74 | 47 | 36 | 29 | 17 | 11 | 8 | 7 |

*Tabelle 5*

| Nichtionisches oberflächenaktives Mittel | Puffer | Che-Stabilität (%) | | | | | | | |
|---|---|---|---|---|---|---|---|---|---|
| | | 2 d | 4 d | 7 d | 14 d | 21 d | 28 d | 35 d | 42 d |
| Isotridecyläther | Tris pH = 7,6 | 110 | 77 | 75 | 75 | 67 | 67 | 55 | 58 |
| | Imidazol pH = 7,6 | 89 | 62 | 78 | 60 | 53 | 55 | 47 | 47 |
| | Mops pH = 7,0 | 87 | 44 | 73 | 71 | 69 | 69 | 55 | 56 |
| ®Triton X-100 | Tris | 67 | 45 | 63 | 49 | 55 | 55 | 45 | 38 |
| | Imidazol | 102 | 62 | 60 | 50 | 45 | 40 | 35 | 30 |
| | Mops | 79 | 55 | 43 | 45 | 32 | 26 | 17 | 18 |

Ein Vergleich der Werte von Tabelle 5 mit den Werten von Tabelle 2 zeigt, dass erfindungsgemäss die Stabilität bei Raumtemperatur ebenso gut ist wie bei Kühlraumtemperatur.

*Beispiel 4*

Wie in Beispiel 2 beschrieben, wurde die Abhängigkeit der Che-Stabilität von der Mg²⁺-Konzentration untersucht, jedoch bei 4 °C. Alle übrigen Bedingungen entsprechen denen von Beispiel 2.

Die Ergebnisse sind in Tabelle 6 wiedergegeben.

*Tabelle 6*

| Nichtionisches oberflächenaktives Mittel | Mg²⁺-Konz. (mmol) | Che-Stabilität (%) | | | | | | | |
|---|---|---|---|---|---|---|---|---|---|
| | | 2 d | 4 d | 7 d | 14 d | 21 d | 28 d | 35 d | 42 d |
| Isotridecyläther | 0 | 93 | 63 | 54 | 48 | 32 | 28 | 21 | 18 |
| | 10 | 110 | 74 | 69 | 71 | 61 | 61 | 46 | 46 |
| | 50 | 110 | 77 | 75 | 75 | 67 | 67 | 55 | 58 |
| | 100 | 92 | 66 | 72 | 68 | 56 | 70 | 62 | 59 |
| | 150 | 93 | 79 | 81 | 77 | 70 | 70 | 63 | 63 |
| "Triton X-100 | 0 | 58 | 35 | 31 | 25 | 13 | 6 | 2 | 0 |
| | 10 | 71 | 44 | 47 | 34 | 30 | 22 | 22 | 17 |
| | 50 | 67 | 45 | 63 | 49 | 55 | 55 | 45 | 38 |
| | 100 | 97 | 58 | 48 | 26 | 25 | 24 | 26 | 24 |
| | 150 | 95 | 61 | 65 | 52 | 46 | 65 | 52 | 46 |

## Patentansprüche

1. Verfahren zur Stabilisierung wässeriger Lösungen von Cholesterinesterase aus Pseudomonaden, insbesondere in Gegenwart eines oberflächenaktiven Mittels, dadurch gekennzeichnet, dass das Enzym in einem phosphatfreien Puffer gelöst wird, welcher 10 bis 200 mmol pro Liter Magnesiumionen enthält.

2. Verfahren nach Anspruch 1, dadurch gekennzeichnet, dass man 25 bis 150 mmol pro Liter Magnesiumionen zusetzt.

3. Verfahren nach einem der Ansprüche 1 oder 2, dadurch gekennzeichnet, dass man einen Puffer pH 5,0 bis 9,0 verwendet.

4. Verfahren nach Anspruch 3, dadurch gekennzeichnet, dass man einen Puffer von pH 6,5 bis 9,0 verwendet.

5. Verfahren nach einem der vorhergehenden Ansprüche, dadurch gekennzeichnet, dass man als Aktivierungsmittel ein anionisches und/oder nichtionisches oberflächenaktives Mittel zusetzt.

## Claims

1. Process for the stabilisation of aqueous solutions of cholesterol esterase from *Pseudomonas*, especially in the presence of a surface-active agent, characterised in that the enzyme is dissolved in a phosphate-free buffer which contains 10 to 200 mmol per litre of magnesium ions.

2. Process according to Claim 1, characterised in that one adds 25 to 150 mmol per litre of magnesium ions.

3. Process according to Claim 1 or 2, characterised in that one uses a buffer of pH 5.0 to 9.0.

4. Process according to Claim 3, characterised in that one uses a buffer of pH 6.5 to 9.0.

5. Process according to one of the preceding claims, characterised in that as activation agent one adds an anionic and/or non-ionic surface-active agent.

## Revendications

1. Procédé de stabilisation de solutions aqueuses de cholestérolestérase de *Pseudomonas*, en particulier en présence d'un agent tensio-actif, caractérisé en ce que l'enzyme est dissoute dans un tampon exempt de phosphate, qui contient 10 à 200 mmol par litre d'ions magnésium.

2. Procédé suivant la revendication 1, caractérisé en ce qu'on ajoute 25 à 150 mmol par litre d'ions magnésium.

3. Procédé suivant l'une des revendications 1 ou 2, caractérisé en ce qu'on utilise un tampon de pH 5,0 à 9,0.

4. Procédé suivant la revendication 3, caractérisé en ce qu'on utilise un tampon de pH 6,5 à 9,0.

5. Procédé suivant l'une des revendications précédentes, caractérisé en ce qu'on ajoute comme agent d'activation un agent tensio-actif anionique et/ou non ionique.

# FIG.1

# FIG. 2

LAGERUNG [TAGE BEI 25°C]

% RESTAKTIVITÄT

# FIG.3

% RESTAKTIVITÄT

100

50

1    2    3

LAGERUNG [ TAGE BEI 25°C ]